# EUROPEAN PATENT APPLICATION

(11) **EP 3 777 874 A1**
(43) Date of publication of application: **17.02.2021**
(21) Application number: 18913330.9
(22) Date of filing: 04.04.2018
(51) Int. Cl.: A61K 36/8988, A61P 17/14, A61P 17/04

(54) **CHINESE HERBAL MEDICINE COMPOSITION FOR PROMOTING INCREASE OF AMOUNT OF HAIR, PREPARATION METHOD THEREFOR AND USE THEREOF**

(71) Applicant: Liu, Yueshao, New Taipei, Taiwan 241 (TW)
(72) Inventor: Liu, Yueshao, New Taipei, Taiwan 241 (TW)
(74) Representative: Straus, Alexander
(86) International application number: PCT/CN2018/081815
(87) International publication number: WO 2019/191918

(57) **Abstract**

A herbal composition for increasing hair volume comprises a first medicinal material including Lilum, Scutellaria barbata D. Don, Carica papaya Linn, Lonicera japonica, or any combinations thereof; a second medicinal material including Rehmannia glutionsa, Cuscuta chinensis, and Crocus sativus; a third medicinal materials including Platycladus orientalis having its bark of root and leaf combined with Black sesame, Platycladus orientalis having its bark of root and leaf combined with Morus having its fruits, root, leaf, and twig, Purple grape, or any combinations thereof; wherein the first medicinal material weighs 2∼40%, the second medicinal material weighs 2∼40%, and the third medicinal material weighs 20∼96% of the total weight of the herbal composition.

## Description

### FIELD OF INVENTION

The present invention generally relates to a herbal composition, and its manufacturing method and use, and more particularly to the herbal composition for increasing hair growth, reducing hair loss, inhibiting scalp itching, reducing dandruff, and/or improving spiritual health, acuity of sight, and skin texture, and its manufacturing method and use.

### BACKGROUND OF THE INVENTION

The growth of hair needs nutrients from blood. When a person suffers from anemia, there is a risk for the person to lose hair. Since hair is one of the fast-growing parts of human body, the texture of hair will be deteriorated, and the hair will turn into white or even fall from the scalp if the supply of nutrients from blood is insufficient. Hair loss may have the following diagnoses: androgenetic alopecia, alopecia areata, alopecia infected by medicine, telogen effluvium, cicatricial alopecia, and so on. Among these, androgenetic alopecia (male baldness) is the most common cause of hair loss. Particularly, people nowadays are under great stress and pressure, have too much greasy food, excessively blow dry their hairs, and stay up late, which exacerbate the crisis of hair loss. Most of the androgenetic alopecia cases are caused by endocrine disorders, adverse drug reaction, malnutrition, heredity, disease, stress, cancer-related treatment. Both men and women may suffer from androgenetic alopecia.

From the perspective of Chinese and Western medicine, whether the hair can grow well or bad seems to be inseparable from the blood. According to one of the classical Chinese medical collections "Liujie cangxiang, Suwen, Emperor's Classic of Medicine", hair growth is relevant to kidney. In addition, some other traditional Chinese medical collection also disclosed the hair growth is relevant to blood. In addition, other Chinese medical collections also mentioned that "Sufficient blood gives moist hair, and insufficient blood leads to bad hair", so that many traditional Chinese doctors believed that hair is nourished by blood, and the quality of hair depends on one's kidney because "Kidney stores essence, essence can transform blood, and hair will become moist and beautiful when one's kidney is healthy". Therefore, kidney and blood can beautify hair. In the principle of traditional medicine, hair is closely related to kidney and blood, and it is very important to take good care of the condition of our kidney and blood. Young people with sufficient blood usually have thick hair of natural and original color. However, the elderly with insufficient blood usually have thin hair of white color . The problem of hair loss can be improved by paying more attention on the nourishment of our kidney and blood.

At present, there are two drugs approved by U.S. Food and Drug Administration (FDA) that can cure alopecia,. One is Propecia which is administrated orally,, and only men can use this drug. The other one is Regaine which is for external use, and both men and women can use this drug, but the concentrations are different for men and women. The treatment usually takes three to six months in order to stop the hair loss and takes one to two years before the hair starts to grow. However, the oral drugs have side effects such as erectile dysfunction which may cause distress for some men.

The active ingredient of Propecia and Regaine is minoxidil, and some people believe that minoxidil can dilate blood vessels and open up potassium ion channels, so that more oxygen and nutrients are delivered to hair follicles, and promote the hair follicles from a rest period to a growth period. However, other vasodilators do not have the same effect of stimulating hair growth as the minoxidil does. Since the mechanism for minoxidil to stimulate hair growth is uncertain, it hinders the development of related drugs and pharmaceutical preparation.

Thus, there is still a need for a drug or a pharmaceutical preparation with fast and sustained effects, and low side effects.

### SUMMARY OF THE INVENTION

A primary objective of the present invention is to provide a herbal composition for increasing hair volume, comprising: a first medicinal material selected from the group consisting of Lilum, Gastrodia elata, Scutellaria barbata D. Don, Carica papaya Linn (mature fruit of Chaenomeles speciosa (Sweet) Nakai and Chaenomeles sinensis (Thouin) Koehne), Lonicera japonica (scientific name: Lonicera japonica Thunb.), and any combinations thereof; a second medicinal material selected from the group consisting of Rehmannia glutionsa, Cuscuta chinensis, and Crocus sativus, and any combinations thereof; a third medicinal material selected from the group consisting of Platycladus orientalis having its bark of root and leaf, and Morus having its fruit (i.e. Mulberry (scientific name: Morus alba Linn)), root, leaf, and twig, Black sesame (scientific name: Sesamum indicum), Purple grape (scientific name: Vitis romanetii Roman. du Caill. ex Planch.), and any combinations thereof.

In the herbal composition of the present invention, the ratio of the first medicinal material, the second medicinal material, and the third medicinal material is not limited thereto. Preferably, based on the total weight of the herbal composition, the first medicinal material is 2∼40%, the second medicinal material is 2∼40%, and the third medicinal material is 20∼96%. More preferably, based on the total weight of the herbal composition, the first medicinal material is 10∼35%, the second medicinal material is 10∼30%, and the third medicinal material is 35∼80%. Most preferably, based on the total weight of the herbal composition, the first medicinal material is 20∼30%, the second medicinal material is 20∼25%, and the third medicinal material is 45∼60%.

In the embodiment of the present invention, the third medicinal material is Platycladus orientalis having its bark of root and leaf combined with Morus having its fruits, root, leaf, and twig; wherein the weight ratio of Platycladus orientalis having its bark of root and leaf to Morus having its fruits, root, leaf, and twig) is 0.8∼1.2:1, preferably 1:1.

In the embodiment of the present invention, the third medicinal material is Platycladus orientalis having its bark of root and leaf, combined with Morus having its fruits, root, leaf, and twig, and Black sesame; wherein their weight ratio is 1∼4:1∼4:1, preferably 2:2:1.

In the embodiment of the present invention, the third medicinal material is Morus having its fruits, root, leaf, and twig combined with Purple grape; wherein their weight ratio is 0.8∼1.2:1, preferably 1:1.

In the embodiment of the present invention, the third medicinal material is Platycladus orientalis having its bark of root and leaf combined with Morus having its fruits, root, leaf, and twig, and Purple grape, and Black sesame; wherein their weight ratio is 1∼5:1∼5:1∼5:1, preferably 3:3:3:1.

In the embodiment of the present invention, the first medicinal material is Lilum, Gastrodia elata, Scutellaria barbata D. Don, Carica papaya Linn, and Lonicera japonica; wherein their weight ratio is 1:0.8:1:1:1.2. In addition, Lonicera japonica is not only used for treatment, but also used as a natural preservative.

In the embodiment of the present invention, the second medicinal material is Rehmannia glutionsa, Cuscuta chinensis, and Crocus sativus; wherein their weight ratio is 1.5:1:0.5.

In the herbal composition of the present invention, the third medicinal materials can account for 100%. The herbal composition is selected from the group consisting of Platycladus orientalis having its bark of root and leaf, and Morus having its fruit (i.e. Mulberry (scientific name: Morus alba Linn)), root, leaf, and twig, Black sesame (scientific name: Sesamum indicum), Purple grape (scientific name: Vitis romanetii Roman. du Caill. ex Planch.), and any combinations thereof.

The herbal composition of the present invention optionally comprises a diaphoretic medicinal material, a diuretic medicinal material, an antifebrile medicinal material, or a medicinal material for invigorating spleen and eliminating dampness.

The amount of the diaphoretic medicinal material, the diuretic medicinal material, the antifebrile medicinal material, or the medicinal material for invigorating spleen and eliminating dampness added in the herbal composition is not limited. Preferably, the diaphoretic medicinal material is 1∼50 wt%, the diuretic medicinal material is 1∼50 wt%, the antifebrile medicinal material is 1∼70 wt%, the medicinal material for invigorating spleen and eliminating dampness is 1∼40 wt%. More preferably, the diaphoretic medicinal material is 5∼30 wt%, the diuretic medicinal material is 5∼30 wt%, the antifebrile medicinal material is 5∼30 wt%, and the medicinal material for invigorating spleen and eliminating dampness is 2∼30 wt%.

The diaphoretic medicinal material optionally used in the present invention is a conventional one, which is selected from the group consisting of Fermented soybean, Ramulus cinamomi, Perillae folium, Zingiberis rhizome, Ephedrae herba, Allii fistulosi Bulbus, Notopterygii rhizome, and any combinations thereof.

The diuretic medicinal material optionally used in the present invention is a conventional one, which is selected from the group consisting of Alismatis rhizome, Semen plantaginis, Elephantopus scaber, Solanum incanum, Indian buead, Beartiful sweetgum Fruit, and any combinations thereof.

The antifebrile medicinal material optionally used in the present invention is a conventional one, which is selected from the group consisting of Tamarix chinenesis Lour., Black olive, Cassia seed, Flos Eriocauli, Sasagrass, and any combinations thereof.

The medicinal material for invigorating spleen and eliminating dampness optionally used in the present invention is a conventional one, which is selected from the group consisting of Euryaleferox Salisb., Amomum kravanh Piere ex Gagnep, Amomum tsao-ko Crevost et Lemaire, Magnolia officinalis Rehd. Et Wils., Eupatorium fortune Turcz, and any combinations thereof.

In the herbal composition of the present invention, the diuretic medicinal material is preferably Ledebouriella seseloides Wolff extracts, Fermented soybean extracts, Notopterygii rhizome extracts, or any combinations thereof. The diuretic medicinal material is preferably Alismatis rhizome extracts, Armand Clematis Stem extracts, Semen plantaginis extracts, or any combinations thereof. The antifebrile medicinal material is preferably Tree peony bark extracts, Dahurian patrinia herb extracts, or any combinations thereof. The medicinal material for invigorating spleen and eliminating dampness is preferably Semen euryales extracts, Atractlodis rhizome extracts, zingiberaceae plant extracts or any combinations thereof.

The aforementioned medicinal material can be Chinese herbal medicine, Chinese herbal medicine extracts, or any combinations thereof. If said medicinal material is Chinese herbal medicine extracts, it is water extracts, ethanol extracts, or water extracts and ethanol extracts. More preferably, the Chinese herbal medicine extracts are water extracts and ethanol extracts. The Chinese herbal medicine extracts are made by spray drying or freeze drying.

The term "increasing hair volume" means that the effect is achieved by promoting hair growth and/or reducing hair loss. Preferably, the hair growth means the hair grows into natural and original color hair, but is not limited thereto. The herbal composition of the present invention delays aging process and boosts metabolism. The white hair drops naturally when it enters into rest period. Then, the new hair should be natural and original color. Furthermore, the herbal composition of the present invention increases the ratio of natural and original color hairs (for example, if the natural and original color is black, the ratio of black hair is increased), inhibits scalp itching, balances scalp fat, and eliminates peculiar smell, and/or reduces dandruff. As used in the specification, natural and original color hair means the color before the hair becomes white, such as black, brown, gold, red, and so on. In addition, the herbal composition of the present invention also has effects of improving the problem of spiritual health, acuity of sight, and/or skin texture.

The herbal composition of the present invention is administered by means of topical dosage form.

The herbal composition of the present invention can be prepared as an oral preparation, and other biocompatible carriers can be added if required. For example, the herbal composition used in oral dosage comprises a capsule, a lozenge, an emulsifier, a suspension, a dispersant, and a solvent. Take lozenge as an example, the conventional carrier is lactose or corn starch. Lubricant or Magnesium Stearate is a basic additive. Take soft capsule as another example, lactose or dried corn starch can be an effective diluent. If required, a sweetener and a flavoring agent can be added optionally. The present invention preferably comprises an excipient. Any conventional one such as corn starch can be used appropriately in the present invention.

The present invention further provides a health drink which can work on germination (i.e. hair growth), hair nourishment, and hair care. The health drink comprises the herbal composition of the present invention, water, and some juice or fruit extracts. In the embodiment of the present invention, the herbal composition is a distillate produced by mixing or made individually by the first, second, and third medicinal materials. The water is pure water or sterilized water, and the fruit is mulberry or grape. Furthermore, each ingredient of the health drink has a weight percentage (based on the total weight of the health drink) as listed in Table 1.

**Table 1. Exemplary embodiment of healthy drink**

| | Pure water | Mulberry extract or Grape extract | Herbal composition (mixed distillate) |
|---|---|---|---|
| Exemplary embodiment 1 | 40∼60% | 0 | 40∼60% |
| Exemplary embodiment 2 | 40∼60% | 10∼15% | 25∼75% |
| Exemplary embodiment 3 | 45∼55% | 10∼15% | 30∼45% |
| Exemplary embodiment 4 | 30∼60% | 15∼20% | 25∼75% |

In another embodiment of the present invention, a natural flavoring agent can be added into the aforementioned healthy drink to improve the drinking quality. For example, 0.1-1 c.c. longan honey is added into 1000 c.c. of the aforementioned healthy drink.

The present invention also provides a method of manufacturing the aforementioned herbal composition, comprising the steps of: (a) providing a first medicinal material, a second medicinal material, and a third medicinal material respectively; (b) mixing the first medicinal material, the second medicinal material, and the third medicinal material to produce a mixture, wherein based on the total weight of the herbal composition, the first medicinal material is 2∼40%, the second medicinal material is 2∼40%, and the third medicinal material is 20∼96%; (c) extracting the mixture; wherein the first medicinal material is selected from the group consisting of Lilum, Gastrodia elata, Scutellaria barbata D. Don, Carica papaya Linn, Lonicera japonica, and combinations thereof; the second medicinal material is selected from the group consisting of Rehmannia glutionsa, Cuscuta chinensis, and Crocus sativus, and combinations thereof; the third medicinal material selected from the group consisting of Platycladus orientalis having its bark of root and leaf, Morus having its fruits, root, leaf, and twig, Black sesame, Purple grape, and combinations thereof.

In addition, the herbal composition can be obtained by mixing the aforementioned medicinal materials in different ratios and then extracting separately, extracting simultaneously, and concentrating the mixed medicinal materials.

The method can be used as a preparation for germination, increasing hair volume, increasing the ratio of natural and original color hairs, inhibiting scalp itching and/or reducing dandruff. As used in the specification, the term "increasing hair volume" means promoting hair growth, reducing hair loss, or stimulating hair growth and reducing hair loss.

In the embodiment of the present invention, the preparation is administered by means of topical dosage form or oral dosage form.

In the embodiment of the present invention, when the preparation is administered by oral dosage form, the preparation additionally comprises a patchouli extract.

In the embodiment of the present invention, the solvent used in the preparation is water or essential oil.

In the embodiment of the present invention, Lonicera japonica used in the herbal composition is a natural preservative.

Many of the attendant features and advantages of the present invention will become better understood with reference to the following detailed description considered in connection with the accompanying drawings.

### BRIEF DESCRIPTION OF THE DRAWINGS

FIGS. 1A to 1B are pictures illustrating conditions before and after the formula A2 is applied on Mr. Chih's head respectively. FIGS. 1A and 1B show the area of hair distribution, and the area of alopecia in FIG. 1B being less than half of that in FIG. 1A respectively.
FIGS. 2A to 2C are pictures that illustrate the condition after formula A2 is applied on Mr. Huang's vertex and hindhead.
FIGS. 3A to 3D are pictures that illustrate the condition after formula A3 is applied on Ms. Liu's forehead.
FIGS. 4A to 4H are pictures that illustrate the condition after formula A4 is applied on Ms. Lin's head; in particular, FIGS. 4G and 4H illustrate the condition after formula A4 is applied on Ms. Lin's hindhead.
FIGS. 5A to 5D are pictures that illustrate the condition after formula A4 is applied on Ms. Liao's head.
FIGS. 6A to 6M are pictures that illustrate the conditions before and after the formula A5 is applied on Mr. Wang's head.
FIGS. 7A to 7M are pictures that illustrate the conditions before and after the formula A5 is applied on Mr. Tsai's head, and the improvement made on his head.
FIGS. 8A to 8G are pictures that illustrate before and after the formula A5 is applied on Mr. Huang's head, and the improvement made on his head.
FIGS. 9A to 9F are pictures that illustrate before and after the formula A5 is applied on Ms. Cheng's head, and the improvement made on her head.

### DETAILED DESCRIPTION OF THE PREFERRED EMBODIMENTS

The manufacturing process of the present invention is conventional in the field of Scientifically Processed Chinese Herbal Medication, the person having ordinary skill in this art can prepare it and extract it by ethanol, water, or combinations thereof according to characteristics of medicinal materials. The extracts extracted from medicinal materials are configured as five formulae (see Table 2) in light of different ratios.

**Table 2. The formula used for embodiments**

| Formula | First medicinal material (weight percentage) | Second medicinal material (weight percentage) | Third medicinal material (weight percentage) |
|---|---|---|---|
| A1 | Lilum: Gastrodia elata: Scutellaria barbata D. Don: Carica papaya Linn: Lonicera japonica =1:0.8:1:1:2 (30%) | Rehmannia glutionsa: Cuscuta chinensis: Crocus sativus =1:5:1:0.5 (20%) | *Mulberry (50%) |
| A2 | Lilum: Gastrodia elata: Scutellaria barbata D. Don: Carica papaya Linn: Lonicera japonica =1:0.8:1:1:1.2 (20%) | Rehmannia glutionsa: Cuscuta chinensis: Crocus sativus =1:5:1:0.5 (25%) | *Platycladus orientalis: *Mulberry =1:1 (55%) |
| A3 | Lilum: Gastrodia elata: Scutellaria barbata D. Don: Carica papaya Linn: Lonicera japonica =1:0.8:1:1:1.2 (20%) | Rehmannia glutionsa: Cuscuta chinensis: Crocus sativus =1:5:1:0.5 (20%) | *Mulberry: *Platycladus orientalis: Black sesame =2:2: 1 (60%) |
| A4 | Lilum: Gastrodia elata: Scutellaria barbata D. Don: Carica papaya Linn: Lonicera japonica =1:0.8:1:1:1.2 (20%) | Rehmannia glutionsa: Cuscuta chinensis: Crocus sativus =1:5:1:0.5 (20%) | Purple grape: *Mulberry =1:1 (60%) |
| A5 | Lilum: Gastrodia elata: Scutellaria barbata D. Don: Carica papaya Linn: Lonicera japonica =1:0.8:1:1:1.2 (30%) | Rehmannia glutionsa: Cuscuta chinensis: Crocus sativus =1:5:1:0.5 (20%) | *Platycladus orientalis: *Mulberry: Purple grape: Black sesame =3:3:3:1 (50%) |

| | | | |
|---|---|---|---|
| *Platycladus orientalis includes its bark of root and leaf *Mulberry includes its root, leaf, and twig | | | |

The amount of each spray is 0.25 ml, at least 3 ml-5 ml are sprayed every day, and there are four times of sprays per day (morning, noon, evening, and before going to bed). If those formulae cannot be applied at the designated time, the application of the formulae can be less than four times. Users use it for three months, and the testing result is evaluated by the spraying amount and the user's feeling. Their total spraying amount should be identical. In order to quantify the testing result, it is graded from zero point to five points, and the factors of anti-hair loss, new hair growth, new hair having natural and original color, acuity of sight, spiritual condition, inhibiting itching and dandruff are used as observation index.
Grading System
Zero point: no improvement
One point: feel a little bit improvement, but not very sure
Two point: improvement for sure
Three point: appropriate improvement
Four point: significant improvement
Five point: extremely significant improvement and others can tell the differences between before and after having this healthy drink

### Embodiment 1: Control Group

Five volunteer testers(users) were chosen for the test in this embodiment, and they kept taking the Mulberry extract for three months continuously, and statistics (as listed in Table 3) were compiled after the testers had finished the test. Observation indexes include anti-hair loss effect, new hair growth, new hair having natural and original color, acuity of sight, spiritual condition, inhibiting itching and dandruff and the average scores of these indexes are 0.8, 1, 1, 0.6, 0.6, and 0.2 respectively. Thus, the testing result falls within the range of "feel a little bit improvement, but not very sure". The total amount of Mulberry extract is 270 ml∼450 ml (3ml∼5ml per day) during three months, but the effect was insignificant. The use of Mulberry extract together with other medicinal compositions should be taken into consideration to have a better effect.

**Table 3**

| Formula | User | Age | Weight (Kg) | Anti-hair loss | New hair growth | New hair having natural and original color | Acuity of sight | Spiritual condition | Inhibiting itching and dandruff |
|---|---|---|---|---|---|---|---|---|---|
| Mulberry | Mr. Lu | 44 | 65 | 0 | 1 | 1 | 1 | 0 | 0 |
| Mulberry | Mr. Lee | 48 | 72 | 1 | 0 | 1 | 1 | 0 | 1 |
| Mulberry | Mr. Huang | 65 | 75 | 2 | 2 | 1 | 2 | 2 | 0 |
| Mulberry | Mr. Liao | 54 | 66 | 0 | 1 | 1 | 0 | 1 | 0 |
| Mulberry | Mr. Chih | 59 | 78 | 1 | 1 | 1 | 0 | 0 | 0 |
| Average score | | | | 0.8 | 1 | 1 | 0.6 | 0.6 | 0.2 |

### Embodiment 2: testing the effect of formula 1

Five users were tested in this embodiment. They used formula A1 of the present invention (as shown in Table 2) for two months continuously, and the total amount of formula A1 was 150∼300 ml. The statistic table was made after they had finished the test (as shown in Table 4). The factors of anti-hair loss, new hair growth, new hair having natural and original color, acuity of sight, spiritual condition, inhibiting itching and dandruff were used as observation index, and the average scores were 3.8, 3.0, 1.8, 2.6, 2.6, and 1.8 respectively. Wherein, the anti-hair loss effect was the most significant, and other factors were also better than those of Embodiment 1. This Embodiment proved that the formula A1 of the present invention had better effect than the conventional Mulberry extract.

**Table 4**

| Formula | User | Age | Weight (Kg) | Anti-hair loss | New hair growth | New hair having natural and original color | Acuity of sight | Spiritual condition | Inhibiting itching and dandruff |
|---|---|---|---|---|---|---|---|---|---|
| A1 | Mr. Huang | 65 | 64 | 5 | 4 | 2 | 3 | 3 | 2 |
| A1 | Mr. Jhou | 28 | 68 | 4 | 2 | 1 | 2 | 3 | 2 |
| A1 | Mr. Peng | 37 | 76 | 3 | 2 | 2 | 3 | 3 | 1 |
| A1 | Mr. Shih | 45 | 60 | 4 | 3 | 2 | 3 | 3 | 2 |
| A1 | Ms. Liu | 50 | 55 | 3 | 4 | 2 | 3 | 3 | 2 |
| Average score | | | | 3.8 | 3.0 | 1.8 | 2.6 | 2.6 | 1.8 |

### Embodiment 3: testing the effect of formula A2

Six users were tested in this embodiment. They used formula A2 of the present invention (as shown in Table 2) for two months continuously, and the total amount of formula A2 was 150∼300 ml. The statistic table was made after they had finished the test (as shown in Table 4). The factors of anti-hair loss, new hair growth, new hair having natural and original color, acuity of sight, spiritual condition, inhibiting itching and dandruff were used as observation index, and the average scores were 4.3, 3.8, 2, 2.7, 2.8, and 2.4 respectively. Wherein, the anti-hair loss effect was the most significant, and other factors were also better than those of Embodiment 1. This Embodiment proved that the formula A2 of the present invention had better effect than the conventional Mulberry extract.

**Table 5**

| Formula | User | Age | Weight (Kg) | Anti-hair loss | New hair growth | New hair having natural and original color | Acuity of sight | Spiritual condition | Inhibiting itching and dandruff |
|---|---|---|---|---|---|---|---|---|---|
| A2 | Ms. Liu | 50 | 65 | 4 | 5 | 3 | 4 | 4 | 2 |
| A2 | Mr. Lu | 44 | 65 | 4 | 3 | 2 | 4 | 4 | 1 |
| A2 | Mr. Lee | 48 | 72 | 4 | 3 | 1 | 2 | 2 | 1 |
| A2 | Mr. Huang | 65 | 66 | 5 | 4 | 3 | 3 | 2 | 2 |
| A2 | Mr. Chih | 59 | 78 | 5 | 4 | 2 | 2 | 1 | 3 |
| A2 | Mr. Chuang | 50 | 75 | 4 | 4 | 1 | 1 | 1 | 3 |
| Average score | | | | 4.3 | 3.8 | 2 | 2.7 | 2.8 | 2.4 |

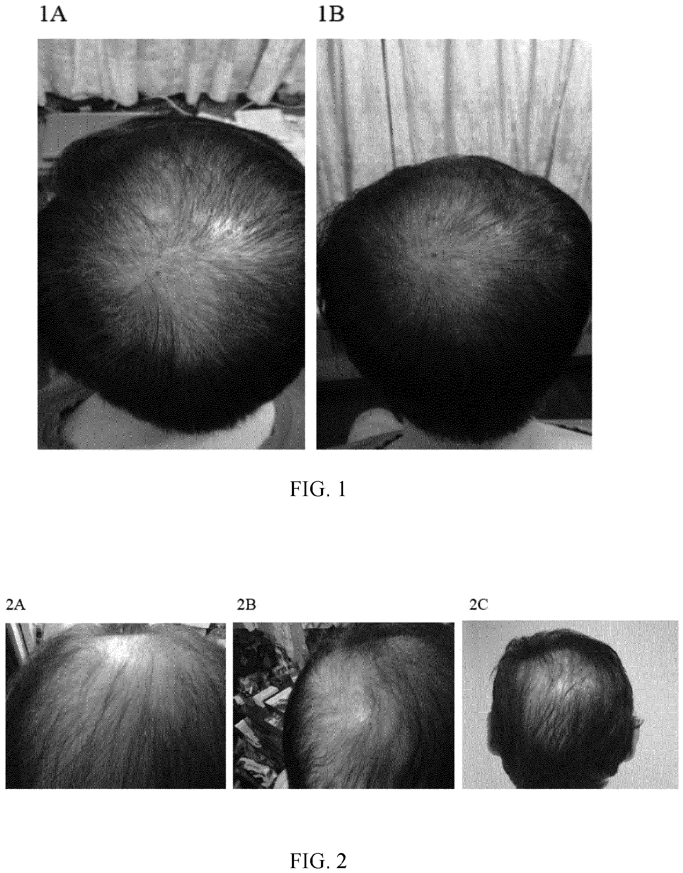

Mr. Chih is 59 years old, and he has suffered from androgenetic alopecia for more than 10 years ago because of unknown reasons. He used Regaine and Propecia for a long time. After applying formula A2 75 ml, new hairs grow 3.5∼4 cm. After applying formula A2 125 ml, some fine hairs grow in the alopecia area and the problem of hair loss is improved. After applying formula A2 150 ml, the fine hairs become thicker and new hairs grow 6∼7.8 cm. The problem of scalp itching is improved, and the amount of dandruff is reduced (as shown in FIG. 1A and FIG. 1B)

Mr. Huang is 65 years old, and he suffered from hair loss in vertex and hindhead because of unknown reasons. His hair volume was thinner and thinner. He used Regaine and Propecia for a long time. After applying formula A2 35 ml, the improvement was not obvious in the first week, only few fine hairs grew up (as shown in FIG. 2A and FIG. 2B). After applying formula A2 75 ml, new hairs grew 2∼3 cm. In the third week, 125 ml of the formula A2 was applied, and the new hairs grown on the vertex grew fast. The hair follicles also grew in the hindhead. New hairs grew 8∼9cm (as shown in FIG. 2C). The problem of alopecia was improved.

### Embodiment 4: testing the effect of formula A3

Eight users were tested in this embodiment. They used formula A3 of the present invention (as shown in Table 2) for two months continuously, and the total amount of formula A3 was 150∼300 ml. The statistic table was made after they finished the test (as shown in Table 5). The factors of anti-hair loss, new hair growth, new hair having natural and original color, acuity of sight, spiritual condition, inhibiting itching and dandruff were used as observation index, and the average score was 4.5, 4.125, 3.875, 3.375, 3.5, and 3. Wherein the effect of anti-hair loss and spiritual condition were the most significant, and other factors were also better than those of Embodiment 1. This Embodiment proved that the formula A3 of the present invention has better effect than conventional Mulberry extract.

**Table 6**

| Formula | User | Age | Weight (Kg) | Anti-hair loss | New hair growth | New hair having natural and original color | Acuity of sight | Spiritual condition | Inhibiting itching and dandruff |
|---|---|---|---|---|---|---|---|---|---|
| A3 | Mr. Chang | 27 | 62 | 5 | 4 | 3 | 3 | 3 | 2 |
| A3 | Mr. Chen | 42 | 69 | 3 | 4 | 2 | 3 | 4 | 3 |
| A3 | Mr. Lee | 33 | 75 | 5 | 5 | 4 | 3 | 3 | 3 |
| A3 | Mr. Huang | 65 | 64 | 5 | 4 | 4 | 4 | 4 | 3 |
| A3 | Mr. Chou | 28 | 68 | 4 | 3 | 3 | 4 | 2 | 2 |
| A3 | Mr. Peng | 55 | 76 | 4 | 4 | 4 | 3 | 3 | 3 |
| A3 | Mr. Shih | 45 | 60 | 5 | 4 | 3 | 3 | 3 | 4 |
| A3 | Ms. Liu | 50 | 55 | 5 | 5 | 4 | 4 | 4 | 4 |
| Average score | | | | 4.5 | 4.125 | 3.875 | 3.375 | 3.5 | 3 |

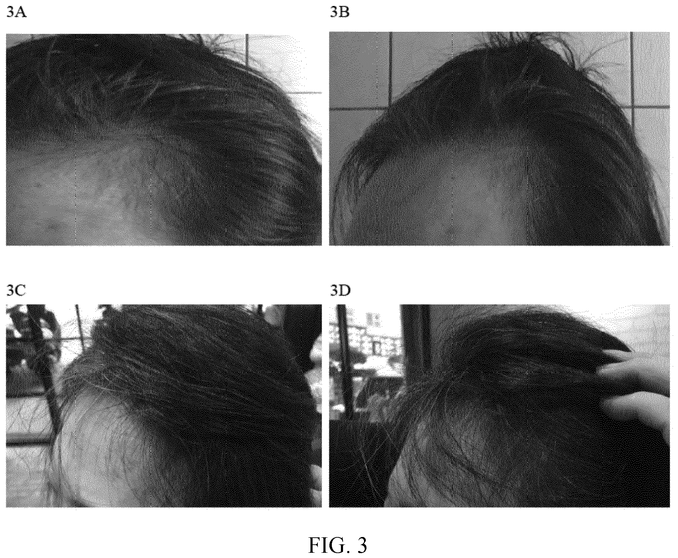

Ms. Liu is 50 years old, and she suffered from hair loss before six years because of surgical treatment of leiomyoma. The hair line became more width. The hair in the area of alopecia became thinner, and fell when it grew up to 1∼2 cm. After applying formula A3 50 ml, she felt the problem of hair loss, scalp itching, and dandruff was improved. After applying formula A3 100 ml, she felt her thin hairs on her head became more thick, and some new hairs also grew up to 3∼4 cm. Her hair on hair line also became thick. After applying formula A3 150 ml, the amount of hair loss was reduced continuously, and the hair volume was thicker. Spiritual condition was also better. Please see FIGS. 3A∼3D, which illustrate before and after the formula A3 150 ml (5 ml per day) was applied on her head and forehead.

### Embodiment 5: testing the effect of formula A4

Eight users were tested in this embodiment. They used formula A4 of the present invention (as shown in Table 2) for two months continuously, and the total amount of formula A4 was 150∼300 ml. The statistic table was made after they finished the test (as shown in Table 6). The factors of anti-hair loss, new hair growth, new hair having natural and original color, acuity of sight, spiritual condition, inhibiting itching and dandruff were used as observation index, and the average score was 3.8, 4, 4.28, 3.2, 4, and 3.3. Wherein the effect of anti-hair loss and spiritual condition were the most significant and other factors were also better than those of Embodiment 1. This Embodiment proved that the formula A4 of the present invention had better effect than conventional Mulberry extract.

**Table 7**

| Formula | User | Age | Weight (Kg) | Anti-hair loss | New hair growth | New hair having natural and original color | Acuity of sight | Spiritual condition | Inhibiting itching and dandruff |
|---|---|---|---|---|---|---|---|---|---|
| A4 | Mr. Wang | 54 | 64 | 3 | 3 | 5 | 2 | 3 | 2 |
| A4 | Mr. Tsai | 24 | 76 | 5 | 3 | 4 | 2 | 4 | 3 |
| A4 | Mr. Huang | 35 | 77 | 4 | 5 | 4 | 3 | 4 | 3 |
| A4 | Mr. Qiu | 60 | 79 | 3 | 5 | 5 | 2 | 3 | 3 |
| A4 | Ms. Liao | 58 | 63 | 4 | 5 | 4 | 2 | 3 | 2 |
| A4 | Ms. Lin | 26 | 55 | 5 | 4 | 5 | 3 | 2 | 3 |
| A4 | Mr. Kao | 38 | 69 | 3 | 4 | 3 | 2 | 4 | 4 |
| Average score | | | | 3.8 | 4.1 | 4.28 | 3.2 | 4 | 3.3 |

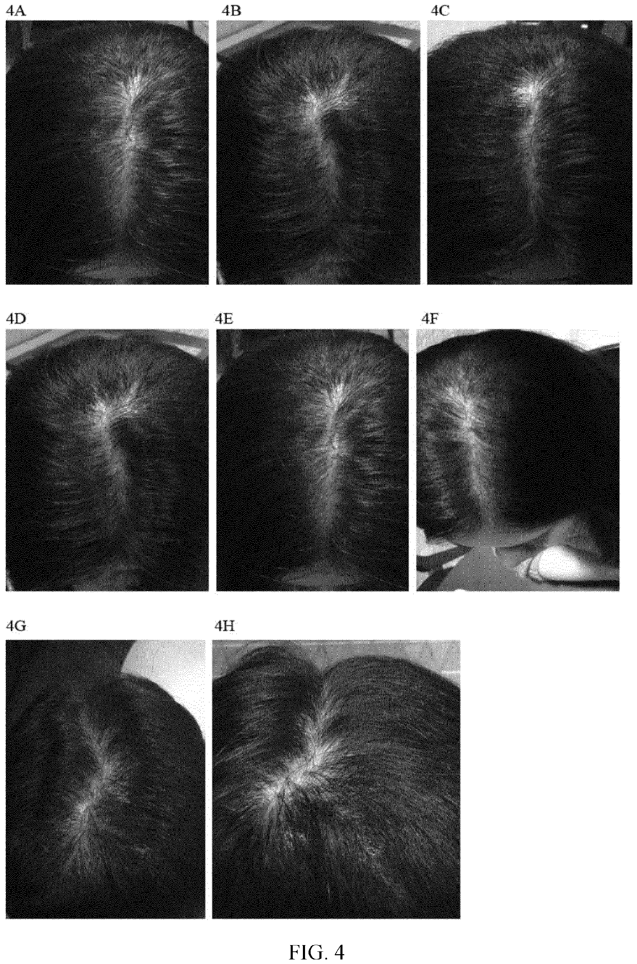

Ms. Lin is 26 years old, and she suffered from hair loss before four years because of menstrual disorder caused by the problem of ovarian. However, after treatment of ovarian problem, her hairs still never grew up, and her hairline seam became thicker. After diagnosing by Hair Care Center, she was told that her hairs never grow up caused by atrophy of hair follicle. After applying formula A4 100 ml (20 days, 5 ml per day), FIGS. 4A∼4F and FIG. 4G∼4H exhibit that her problem of hair loss was improved.

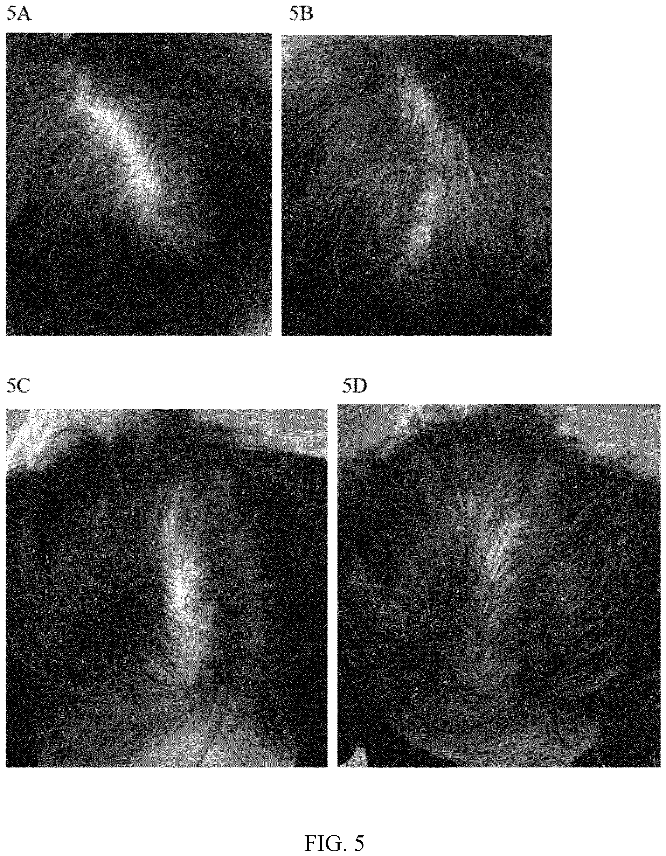

Ms. Liao is 58 years old, and her hairline seam became thicker. After applying formula A4 50 ml (10 days, 5 ml per day), FIGS. 5A and 5B illustrate that the new hairs grew up to 5∼6 cm, but the hair thickness should be enhanced. FIGS. 5C and 5D illustrate that said problem was improved, and her hair line was reduced. Ms. Liao says the amount of hair loss was reduced, and the effect was good although only few amount of formula A4 is applied on her.

### Embodiment 6: testing the effect of formula A5

Eight users were tested in this embodiment. They used formula A5 of the present invention (as shown in Table 2) for two months continuously, and the total amount of formula A4 is 150∼300 ml. The statistic table was made after they finished the test (as shown in Table 6). The factors of anti-hair loss, new hair growth, new hair having natural and original color, acuity of sight, spiritual condition, inhibiting itching and dandruff were used as observation index, and the average score was 4.375, 4.375, 4.625, 3.875, 3.5, and 3.75. Wherein the effect of anti-hair loss and spiritual condition were the most significant and other factors were also better than those of Embodiment 1. This Embodiment proved that the formula A5 of the present invention had better effect than conventional Mulberry extract.

**Table 8**

| Formula | User | Age | Weight (Kg) | Anti-hair loss | New hair growth | New hair having natural and original color | Acuity of sight | Spiritual condition | Inhibiting itching and dandruff |
|---|---|---|---|---|---|---|---|---|---|
| A5 | Mr. Tsai | 65 | 72 | 2 | 4 | 4 | 4 | 4 | 4 |
| A5 | Ms. Cheng | 53 | 68 | 5 | 5 | 5 | 5 | 4 | 4 |
| A5 | Mr. Chen | 24 | 75 | 5 | 4 | 5 | 3 | 3 | 3 |
| A5 | Mr. Huang | 65 | 75 | 5 | 4 | 5 | 3 | 2 | 4 |
| A5 | Ms. Lin | 53 | 71 | 4 | 5 | 5 | 4 | 3 | 4 |
| A5 | Mr. He | 40 | 58 | 5 | 5 | 4 | 4 | 4 | 3 |
| A5 | Mr. Chou | 41 | 67 | 5 | 4 | 4 | 5 | 4 | 4 |
| A5 | Mr. Wang | 54 | 68 | 4 | 4 | 5 | 3 | 4 | 4 |
| Average score | | | | 4.375 | 4.375 | 4.625 | 3.875 | 3.5 | 3.75 |

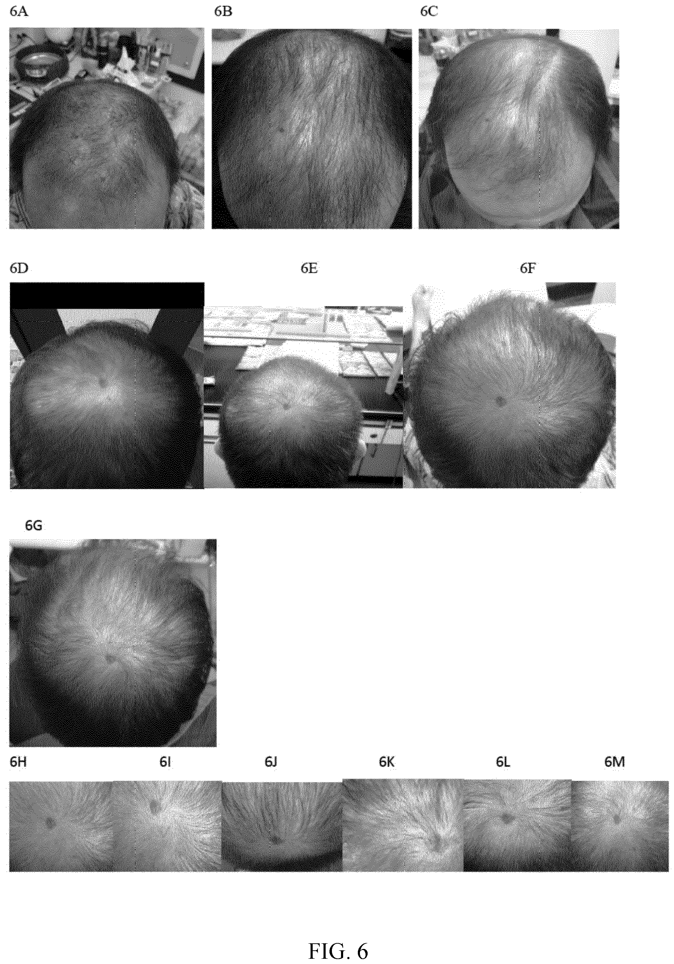

Mr. Wang is 54 years old, and his hair became thinner gradually ten years ago since his hair did not grow and he was suffered from hair loss. His hair was thin in vertex, and did not grow further when the hair grew up to a certain length. His hindhead became alopecia, and he was diagnosed as androgenetic alopecia by the Hair Care Center. After applying formula A5 100 ml, he felt that he slept well, and his spiritual condition was better. His hair loss, scalp itching, and dandruff were reduced. His hair follicle started to grow up eventually, and his hairs grew up to 7∼8 cm. FIGS. 6A∼6C illustrate the conditions before and after 100 ml of the formula A5 was applied and after 250 ml of the formula A5 was applied (for 50 days) respectively. FIGS. 6D∼6G illustrate his hair in hindhead, wherein FIG. 6D illustrates the condition before formula A5 was applied, and FIGS. 6E∼6G respectively illustrate the results after75ml of the formula A5 (15 days), 100ml (20 days), and 200 ml (40 days) are applied. FIGS 6H∼6M illustrate his hair growth condition.

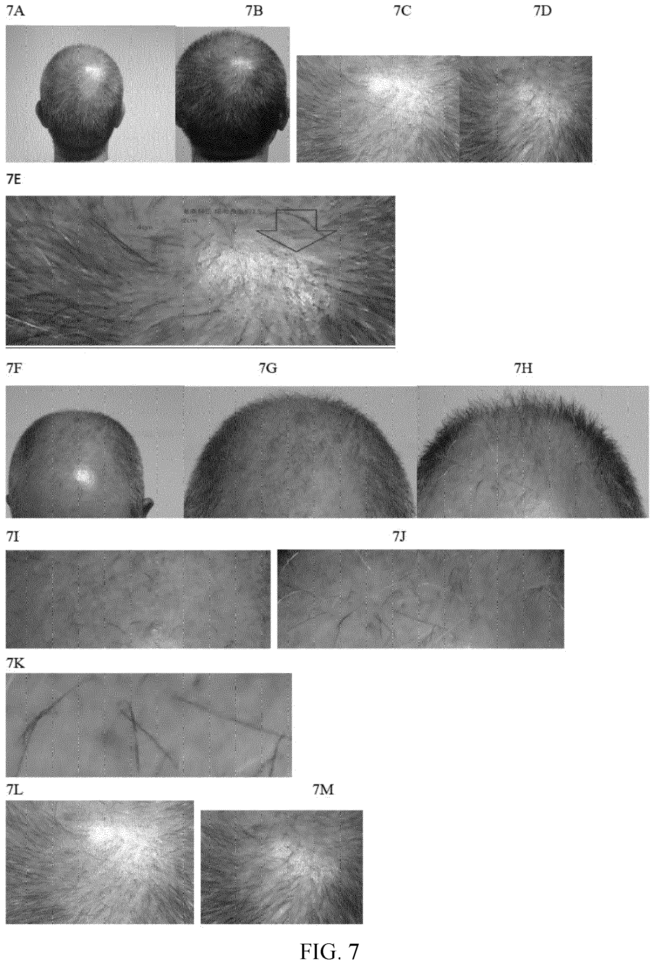

Mr. Tsai is 65 years old, and his hair became thinner gradually twenty years ago. There is almost no hair on his head, but many fine hairs can be detected by hair detector. He still looked alopecia since those fine hairs never grow up but fall off from his scalp. This is a typical symptom of alopecia areata and androgenetic alopecia. Please compare FIG. 7A with FIG. 7B, and the comparison of FIG. 7A with FIG. 7B illustrates that the hairs in his hindhead grew faster. After applying 75 ml of formula A5 (for 15 days), Mr. Tsai said the amount of his white hairs is reduced, and some black hairs started to grow up (as shown in FIGS. 7C∼7E). After applying 50 ml of formula A5, his spiritual condition became better, and some hairs also started to grow up (FIG. 7F illustrates the condition before the formula A5 is applied; FIG. 7G and 7H illustrate new hairs grew up and then his hairs did not fall; FIG. 7K illustrates that new black hairs grew). In addition, after applying 75 ml of formula A5, the thin hair apparently became thicker. It means that the growth period of his hair extended, and thus his hair did not fall. FIG. 7L illustrates the schematic picture of alopecia, and FIG. 7M illustrates new black hairs starting to grow up and the hair follicle was activated to grow few fine hairs. It reveals that the herbal composition of the present invention has the effect of repairing and recovering hairs.

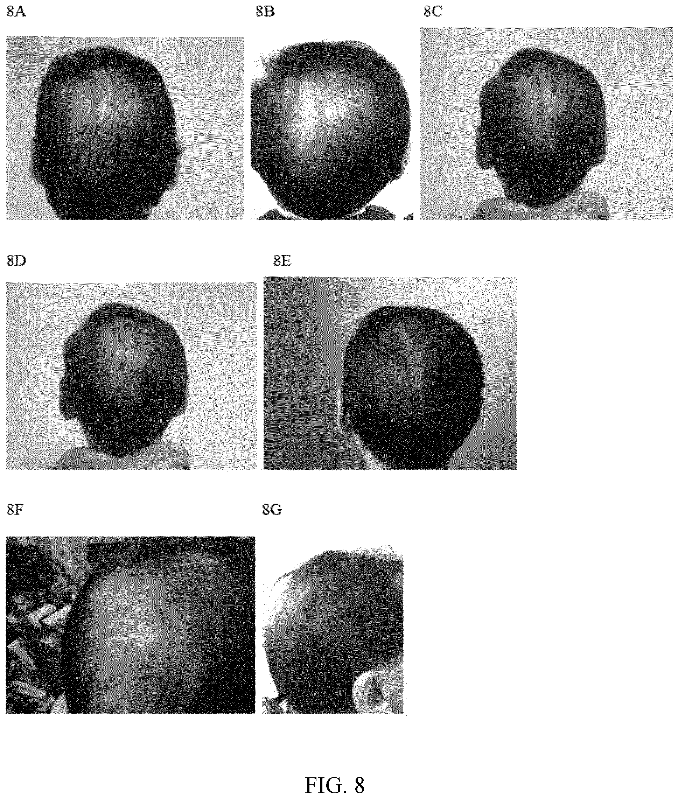

Mr. Huang is 65 years old, and his hair started to grow up after applying formula A3. However, his hair follicles atrophied seriously so that the concentration of hairs was not high enough. He was further suggested to use formula A5. After applying 50 ml of the formula A5 (for 10 days), the results were shown in FIGS. 8A∼8E. FIG. 8C illustrates the new hair started to grow up at location of hair follicles atrophied, and new hairs grew up continuously and did not fall. The area of alopecia was smaller than half. Please see FIGS. 8F and 8G, the thin hairs on the top of his head became thicker. Please see FIG. 8E, the length of hairs is more than 15 cm, and the hairs can be combed to his hindhead.

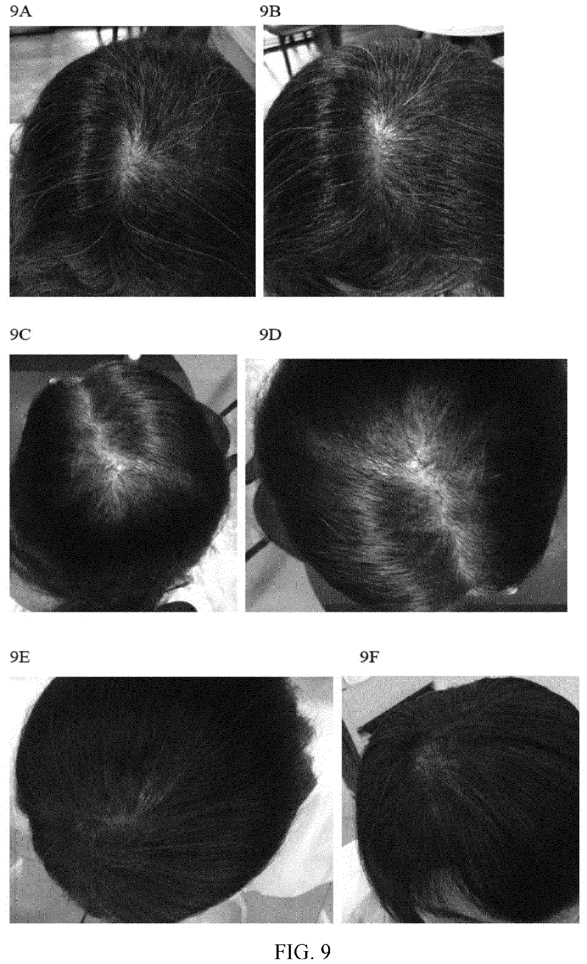

Ms. Cheng is 53 years old, and the hair line of her white hair became thicker and thicker. Her hair follicles were atrophied due to high working pressure (please see FIGS. 9A and 9B). After 75 ml of the formula A5 was used (for 15 days), her new black hairs started to grow up, the amount of hair loss is reduced, and her hairs became thicker (please see FIGS. 9C∼9F).

From those test results, the herbal composition of the present invention not only reduces hair loss, stimulates hair growth into natural and original color, but also improves spiritual condition and skin texture. Furthermore, the effect and function are much better than those of the conventional Mulberry, Black sesame, and Purple grape used individually.

In addition, it should be noted that the aforementioned embodiments are merely exemplary examples, but not intended for limiting the scope of the present invention. For example, the third medicinal material in the Table 2 can be 100%, and the main components of the third medicinal material are Platycladus orientalis having its bark of root and leaf combined with Black sesame, Platycladus orientalis having its bark of root and leaf combined with Morus having its fruits, root, leaf, and twig, Purple grape, or any combinations thereof.

It will be understood that the above description of embodiments is given by way of example only and that various modifications may be made by those with ordinary skill in the art. The above specification, examples, and data provide a complete description of the present invention and use of exemplary embodiments of the invention. Although various embodiments of the invention have been described above with a certain degree of particularity, or with reference to one or more individual embodiments, those with ordinary skill in the art could make numerous alterations or modifications, such as polymers which have similar characteristics with polyacrylate, to the disclosed embodiments without departing from the spirit or scope of this invention.

## Claims

1. A herbal composition for increasing hair volume, comprising:
a first medicinal material including Lilum, Gastrodia elata, Scutellaria barbata D. Don, Carica papaya Linn, Lonicera japonica, or any combinations thereof;
a second medicinal material being as Crocus sativus;
a third medicinal material including Platycladus orientalis having its bark of root and leaf, Black sesame, Morus having its fruits, root, leaf, and twig, Purple grape, or any combinations thereof;
wherein the first medicinal material is 2∼40%, the second medicinal material is 2∼40%, and the third medicinal material is 20∼96% based on the total weight of the herbal composition.

2. The herbal composition as defined in claim 1, wherein the third medicinal material is Platycladus orientalis having its bark of root and leaf combined with Morus having its fruits, root, leaf, and twig, and the weight ratio of them is 0.8∼1.2:1.

3. The herbal composition as defined in claim 1, wherein the third medicinal material is Platycladus orientalis having its bark of root and leaf combined with Morus having its fruits, root, leaf, and twig, and Black sesame, and the weight ratio of them is 1∼4:1∼4:1

4. The herbal composition as defined in claim 1, wherein the third medicinal material is Morus having its fruits, root, leaf, and twig combined with Purple grape, and the weight ratio of them is 0.8∼1.2:1.

5. The herbal composition as defined in claim 1, wherein the third medicinal material is Platycladus orientalis having its bark of root and leaf combined with Morus having its fruits, root, leaf, and twig, and Purple grape, and Black sesame, and the weight ratio of them is 1∼5:1∼5:1∼5:1.

6. The herbal composition as defined in claim 1, wherein the first medicinal material, the second medicinal material, and the third medicinal material are made from Chinese herbal medicine extract.

7. A method of manufacturing the herbal composition recited from claim 1, comprising following steps:
(a) providing a first medicinal material, a second medicinal material, and a third medicinal material; and
(b) mixing the first medicinal material, the second medicinal material, and the third medicinal material to be a mixture, wherein the first medicinal material is 2∼40%, the second medicinal material is 2∼40%, and the third medicinal material is 20∼96% based on the total weight of the herbal composition; and
(c) extracting the mixture.
wherein the first medicinal material is selected from the group consisting of Lilum, Gastrodia elata, Scutellaria barbata D. Don, Carica papaya Linn, and Lonicera japonica; the second medicinal material is Crocus sativus; the third medicinal material selected from the group consisting of Platycladus orientalis having its bark of root and leaf, and Morus having its fruits, root, leaf, and twig, Black sesame, and Purple grape.

8. The herbal composition as defined in claim 1, wherein the herbal composition is used as a preparation for increasing hair volume.

9. The herbal composition as defined in claim 1, wherein the herbal composition is used as a preparation for increasing the ratio of natural and original color hairs.

10. A herbal composition for increasing hair volume, comprising: Chinese Arborvitae Twig and Leaf having its root, bark, and leaf combined with Black sesame, Chinese Arborvitae Twig and Leaf having its root, bark, and leaf combined with Mulberry having its root, leaf, and twig, Purple grape, or any combinations thereof.
